# EUROPEAN PATENT SPECIFICATION

(11) **EP 1 465 552 B1**
(45) Date of publication and mention of the grant of the patent: **09.05.2007**
(21) Application number: 03703686.0
(22) Date of filing: 03.01.2003
(51) Int. Cl.: A61F 2/82

(54) **PROSTHESIS IMPLANTABLE IN ENTERAL VESSELS**
PROTHESE IMPLANTIERBAR IN DARMGEFÄSSE
PROTHESE IMPLANTABLE DANS DES VAISSEAUX DE L'APPAREIL DIGESTIF

(30) Priority: 04.01.2002 US 38640
(43) Date of publication of application: 13.10.2004
(73) Proprietor: Boston Scientific Scimed, Inc., Maple Grove, MN 55311 (US)
(72) Inventor: STINSON, Jonathan, S., Plymouth, MN 55447 (US)
(74) Representative: Cloughley, Peter Andrew
(86) International application number: PCT/US2003/000181
(87) International publication number: WO 2003/057079

(56) References cited:
- WO-A-01/35864
- FR-A- 2 765 097
- US-A- 5 575 818
- US-A- 5 749 919
- US-A- 5 836 966

## Description

### Background of the Invention

The present invention relates to radially expandable prostheses such as stents and stent-grafts positionable within body lumens, and more particularly to prostheses intended for enteral vessels and other body lumens having natural curvature.

A variety of treatment and diagnostic procedures involve the intraluminal placement and implantation of self-expanding medical prostheses. Such devices are described in U.S. Patent No. 4,655,771 (Wallsten). The Wallsten devices are tubular structures formed of helically wound and braided strands or thread elements. The strands can be formed of body compatible metals, e.g. stainless steels, cobalt-based alloys or titanium-based alloys. Alternatively the strands can be formed of polymers such as PET and polypropylene. In either event the strands are flexible to permit an elastic radial compression of the prosthesis by its axial elongation.

Typically, a catheter or other suitable delivery device maintains the prosthesis in its radially compressed state when used to intraluminally carry the prosthesis to an intended treatment site for release from the catheter. Upon its release, the prosthesis radially self-expands while its axial length becomes shorter.

The prosthesis is designed to engage surrounding tissue before it expands to its free, unstressed state, thus to provide acute fixation by virtue of an elastic restoring force due to a slight radial compression. The stent or other device may cause a slight radial enlargement of the lumen at the treatment site, yet continue to exert a self-fixating radially outward force, so long as its radius remains smaller than the free-state radius.

For many applications, this self-expanding tendency is considered an advantage as compared to balloon-expandable devices, which typically are made of plastically deformable metals. Further as compared to balloon-expandable devices, a self-expanding device can be deployed without a balloon or other expansion apparatus.

Radially self-expanding prostheses can be provided in configurations other than the interbraided helices discussed in the aforementioned Wallsten patent, e.g. a single coil, or a serpentine configuration of alternating loops designed to allow radial self-expansion without any appreciable axial shortening.

In any event, the radially outward acting restoring force exerted by a given device when compressed to a certain radius less than its free-state radius, depends on the nature of the strands and the device geometry. More particularly, larger-diameter strands, a larger number of strands, and strands formed of a metal or other material having a higher modulus of elasticity, result in a higher level of restoring force. In structures employing helical strands, the restoring force can be increased by increasing the strand crossing angle, i.e. by winding the strands at a lower pitch angle.

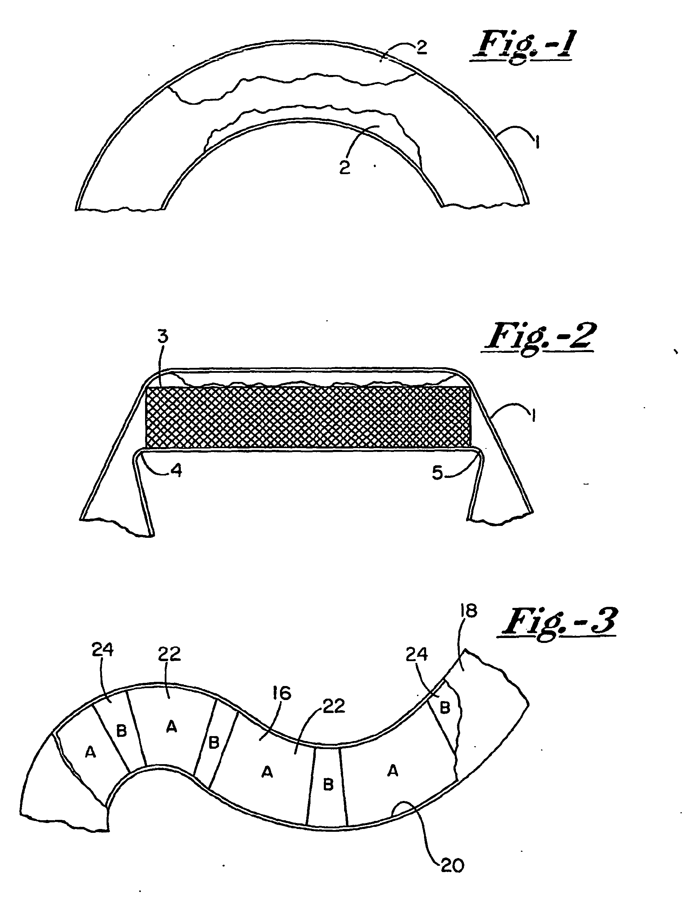

Thus, radially self-expanding prostheses can be tailored to facilitate their fixation in a variety of different types and sizes of body lumens. At the same time, practitioners have encountered problems when using these prostheses in body lumens having natural curvature, such as the colon, the duodenum, the iliac and aortic arch, vena caval arch, brachial arch, and fallopian tubes. To illustrate the problem, Figure 1 schematically shows a curved vessel 1 with an occlusion 2. As seen in Figure 2, a stent 3 has been deployed within vessel 1 to maintain vessel patency, perhaps after an angioplasty balloon has been used to enlarge the vessel in the area of the occlusion. The radial force exerted by stent 3 is a key factor in maintaining vessel patency and in fixing the stent within the vessel.

Figure 2 reveals the tendency of stent 3 to straighten the naturally curved vessel, causing a kinking in the vessel (in this case, the colon) near the ends of the stent, as indicated at 4 and 5. The result is an unwanted narrowing of the vessel, and in the case of severe kinking, an obstruction. The source of this problem is the axial stiffness (lack of axial flexibility) of the stent.

The axial stiffness can be reduced by reducing the diameter of the strands making up the stent, using a strand material with a lower modulus of elasticity, or by reducing the number of strands involved. The trouble with these "solutions" is that each reduces the radially outward restoring force exerted by the stent when engaged with surrounding tissue as shown in Figure 2, with the result that the stent fails to provide the necessary degree of lumen patency and acute fixation.

In connection with prostheses formed of helically wound strands such as stent 3, axial flexibility can be improved by increasing the strand crossing angle of the helices. This approach may appear attractive at first, because the axial stiffness can be reduced without reducing the radially outward restoring force. As noted above, increasing the strand crossing angle increases the radially outward force. The difficulty lies in the fact that as the strand crossing angle increases, so does the extent of stent axial shortening occasioned by a given radial expansion. This increases the need for accurately matching the size of the intended stent with the lumen to be treated, given the increased penalty for excessive radial expansion of the stent once deployed. A related problem is the reduced tolerance for error in axially positioning the stent before its release from the catheter or other deployment device for radial self-expansion.

Other prior art devices including a single first braided segment and a plurality of second segments with differing stiffness are disclosed in US 5 575 818 and WO 01/35864.

Therefore, it is an object of the present invention to provide a medical device deployable in a curved body lumen to maintain lumen patency, with axially extending segments of the device individually tailored to support either more gradually curved or more severely curved regions of the lumen.

Another object is to provide a process for fabricating a body insertable tubular structure with discrete axially extending segments in an arrangement of segments with a relatively high axial stiffness alternating with segments with a relatively high axial flexibility.

A further object of the invention is to provide a body insertable prosthesis which, when engaging surrounding tissue after its deployment, provides alternating regions varied selectively as to axial flexibility, radial stiffness, or both.

Yet another object is to provide a process for fabricating a body insertable prosthesis to selectively form discrete segments including at least one segment with relatively high radial force and axial stiffness, and at least one segment with a relatively low radial force and axial stiffness.

### Summary of the Invention

According to a first aspect of the invention, there is provided a prosthesis insertable into body lumens with natural curvature, including:
a body insertable tubular wall incorporating a plurality of first braided tubular wall segments and a plurality of second tubular wall segments in an alternating sequence, the first and second tubular wall segments having respective nominal diameters when in a relaxed state and being radially compressible against an elastic restoring force to a predetermined diameter;
wherein the first and second wall segments when radially compressed to the predetermined diameter have respective axial stiffness levels, with the first tubular wall segments having relatively high first axial stiffness levels, and with the second tubular wall segments having second axial stiffness levels lower than the first axial stiffness levels, whereby the second tubular wall segments, as compared to the first tubular wall segments, are adapted to more readily conform to a curvature of a body lumen in which the tubular wall is deployed.

According to another aspect of the present invention, there is provided a body insertable device. The device includes a tubular structure including at least one flexible strand selectively formed to define a plurality of discrete tubular segments of the tubular structure. Along the first segments the strand incorporates a first number of filaments. Along the second segments the strand incorporates a second number of filaments less than the first number. As a result, the tubular structure has a first axial stiffness along the first region, higher than a second axial stiffness along the second region.

The tubular structure can be formed by winding one or more flexible strands into the desired tubular shape, wherein each flexible strand is a composite that includes the first number of filaments along its entire length. Then, at least one of the filaments is removed from the tubular structure along the second region, leaving in place the second number of filaments. The filament removal can be accomplished by selective cutting, selective heating to melt or ablate certain filaments at predetermined points, or by selectively dissolving soluble filaments along the second region. When the filaments are to be selectively heated or dissolved, the composite strand includes different types of filaments, with one type that is susceptible to heating or soluable, and another type that is not.

According to another aspect of the present invention, there is provided a prosthesis insertable into body lumens with natural curvature. The prosthesis includes a body insertable tubular wall incorporating an alternating sequence of a plurality of first and a plurality of second tubular wall segments. Each of the wall segments has a nominal diameter when in a relaxed state and is radially compressible against an elastic restoring force to a predetermined diameter. When compressed to the predetermined diameter, the wall segments have respective axial stiffness levels, with each of the first tubular wall segments having a relatively high axial stiffness level and with each of the second tubular wall segments having an axial stiffness level lower than that of the first tubular wall segments. Thus, the second tubular wall segments, as compared to the first tubular wall segments, more readily conform to a curvature of a body lumen in which the tubular wall is deployed.

According to another aspect of the present invention, there is provided a body insertable stent. The stent includes a stent structure formed of at least one flexible composite strand and adapted for deployment at a site within a body lumen to apply a radially outward force against surrounding tissue at the site. The composite strand includes at least one biostable filament and at least one bioabsorbable filament, and is adapted to apply the radially outward force at an initial level upon deployment arising from a combination of the at least one biostable filament and the at least one bioabsorbable filament. The at least one bioabsorbable filament is absorbable in situ, thereby to reduce the radially outward force toward a second level arising from the at least one biostable filament alone.

The stent structure along its length includes several discrete regions including a plurality of first regions along which the at least one strand incorporates the at least one biostable filament and the at least one bioabsorbable filament, and a plurality of second regions along which the at least one strand incorporates only the at least one biostable filament.

According to another aspect of the present invention, there is provided a process for fabricating a prosthesis insertable into body lumens with natural curvature, including:
braiding at least first and second flexible biocompatible and thermally formable strands about a shaping mandrel to form a tubular wall;
forming along the tubular wall a plurality of first tubular wall segments and a plurality of second tubular wall segments in an alternating sequence, the first and second segments having respective nominal diameters when in a relaxed state and being radially compressible against an elastic restoring force into a reduced-diameter configuration in which the first and second tubular wall segments have a predetermined diameter, the first tubular wall segments have relatively high first axial stiffness levels, and the second tubular wall segments have second axial stiffness levels lower than the first axial stiffness levels for better conformity to curvature along body lumens; and
while maintaining the tubular wall in a selected shape, heating the tubular wall to a temperature sufficient to thermally impart the selected shape to the tubular wall.

The heat-set mandrel can have a uniform diameter, or alternatively can include discrete sections with different diameters.

Further according to the present invention, there is provided a process for fabricating a body insertable prosthesis with segments that differ in axial stiffness and radial force. The process includes:
a. providing a flexible strand that is a composite of at least two body compatible filaments;
b. selectively winding the strand to form a tubular structure with a selected shape; and
c. selectively removing at least one of the filaments from the at least one strand along a plurality of predetermined axially extended regions of the tubular structure, whereby the tubular structure along the predetermined region has a reduced axial stiffness level and a reduced radial force level as compared to a remaining region of the tubular structure.

In accordance with the present invention, a medical device deployable in a curved body lumen to maintain lumen patency includes axially extending segments individually tailored to support either more gradually curved or more severely curved regions of the lumen. Segments with higher axial stiffness can be provided along relatively straight regions of the lumen, with segments having relatively high axial flexibility provided along the more severely curved regions. The devices can incorporate segments with different pitch angles or braid angles, whereby segments with higher axial flexibility also exert higher levels of radial force. The devices can be formed of strands that incorporate different numbers of filaments along different segments, such that the more axially flexible segments exert lower levels of radial force. Strands incorporating several filaments also can incorporate at least one bioabsorbable filament, providing levels of radial force and axial stiffness that decrease in situ.

### In the Drawings

For a further understanding of the above and other features and advantages, reference is made to the following detailed description and to the drawings, in which:
Figure 1 is a schematic view of a curved body vessel with an occlusion;
Figure 2 is a schematic view-of a conventional stent implanted in the vessel;
Figure 3 is a schematic view of a stent, fabricated according to the present invention, implanted in a curved body vessel, a portion of which is cut away to reveal the stent;
Figure 4 is an elevational view of another stent constructed in accordance with the present invention;
Figure 5 is a side elevation of an alternative embodiment stent;
Figure 6 illustrates the stent of Figure 5 implanted in a body vessel;
Figure 7 is a side elevation of a further alternative embodiment stent;
Figure 8 is an enlarged partial view of the stent in Figure 7, showing a single strand of the stent;
Figure 9 is a side elevation of a further alternative embodiment stent;
Figure 10 is a side elevation of the stent shown in Figure 9, after a bioabsorbable constituent of the stent has been absorbed in situ:
Figure 11 is a side elevation of an alternative embodiment stent composed of a single strand;
Figures 12 and 13 illustrate stages in a process for fabricating a stent similar to that shown in Figure 4;
Figures 14 and 15 illustrate stages of a process for fabricating a stent similar to that shown in Figure 5;
Figure 16 illustrates a braiding stage of a process for fabricating a stent similar to that shown in Figure 5;
Figures 17 and 18 illustrate stages in a process for fabricating a stent similar to that shown in Figure 7; and
Figure 19 illustrates a stage of a process for fabricating a stent similar to that shown in Figure 7.

### Detailed Description of the Preferred Embodiments

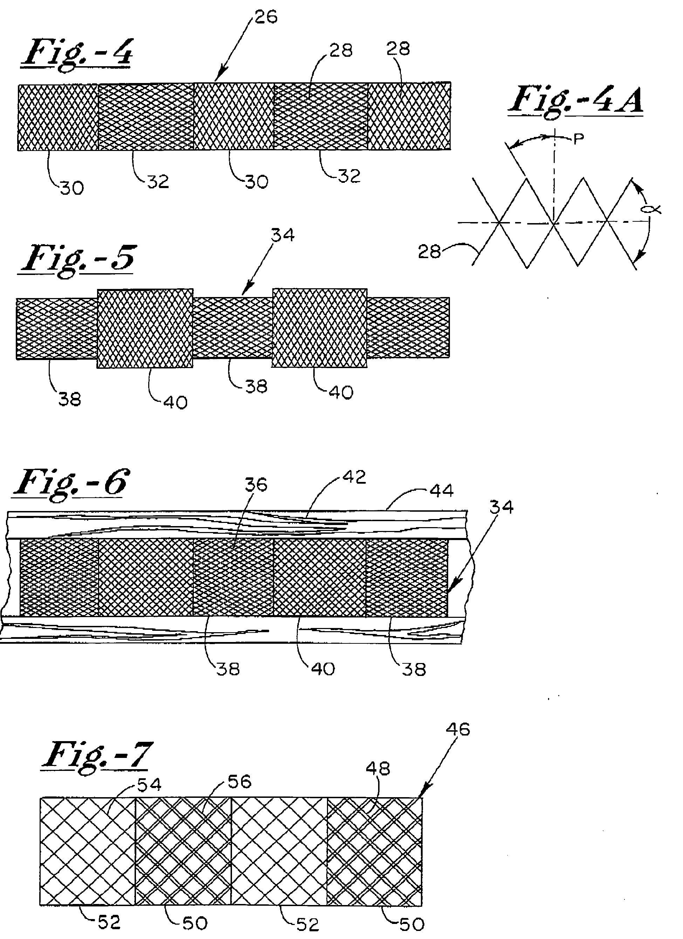

Turning now to the drawings, there is shown in Figure 3 a body insertable stent 16, implanted within a body vessel having natural curvature, in particular the colon 18. The colon has a tissue wall 20 which surrounds the stent over its entire length, although part of the tissue wall is cut away in the figure to reveal the stent.

Stent 16 is a radially self-expanding stent, formed of helically wound, interbraided flexible strands. Consequently the stent is deformable elastically to a reduced-radius, extended-length configuration to facilitate its intraluminal delivery to the treatment site in the colon. Once at the treatment site, the stent is released from a catheter or other delivery device (not shown) and radially self-expands into an engagement with tissue wall 20. When engaged with the wall, stent 16 is maintained at a predetermined diameter less than a diameter of the stent when in a free state, i.e. when subjected to no external stress. Accordingly the stent as shown in Figure 3 retains an internal elastic restoring force, and exerts this force radially outwardly against tissue wall 20. This force acts against obstructive tissue to maintain a patency of the lumen within the colon. The force also tends to embed the stent strands into the vessel wall, and thus prevent stent migration. Meanwhile, tissue wall 20 exerts a counteracting radially inward force to contain the stent.

Stent 16 is composed of a plurality of discrete segments, including two different types of segments 22 and 24 respectively labeled "A" and "B" in the figure. The stent segments have different properties when stent 16 is maintained at the predetermined diameter as shown. In particular, stent segments 22, as compared to stent segments 24, have a higher axial or longitudinal stiffness. Segments 24 have higher axial flexibility, and accordingly more readily conform to the curvature of colon 18. Stent segments 22 mitigate the axial foreshortening of the stent, as the stent radially self-expands after its release from a deployment device.

As seen in Figure 3, segments 22 and 24 are arranged in an alternating sequence. Stent segments 22 are disposed along the straighter, more gradually curved regions of colon 18, while stent segments 24 are positioned along the more severely curved sections of the vessel. Accordingly, segments 22 and segments 24 cooperate to provide the stent fixation force. Segments 24 avoid the kinking problem that arises when a stent with excessive axial stiffness tends to straighten a naturally curved region of the colon or another vessel. Segments 22 serve to dilute the overall stent foreshortening that would complicate positioning of the stent across a lesion during deployment.

As shown in Figure 3, segments can vary as to their lengths. In some applications, segments 22 are longer than segments 24 as shown, while in other applications segments 24 are longer. Although only two types of segments are shown, it is possible to provide segments exhibiting three or more different levels of radial force or axial stiffness.

Figure 4 illustrates an alternative embodiment stent 26 formed by helically winding and interbraiding multiple elastic biocompatible strands 28. The strands as flexible, preferably formed of a cobalt based alloy sold under the brand name Elgiloy. Suitable alternative metals include stainless "spring" steel and titanium/nickel alloys. Certain polymers as also suitable, including PET, polypropylene, PEEK, HDPE, polysulfone, acetyl, PTFE, FEP, and polyurethane.

Stent 26 consists of an alternating sequence of segments 30 in which the strands form a crossing angle of 150 degrees, and segments 32 in which the strands define a crossing angle of 130 degrees. The strand crossing angle or braid angle is conveniently thought of as an angle bisected by a plane incorporating a longitudinal central axis of the stent, i.e. a horizontal axis as viewed in Figure 4. The pitch angle is the angle at which the strands are wound with respect to a plane normal to the axis. Thus, the pitch angles of segments 30 and 32 are 15 degrees and 25 degrees, respectively. In Figure 4a, "p" indicates the pitch angle and "α" indicates the strand crossing angle.

The higher strand crossing angle in segments 30 results in a higher level of radially outward force exerted by these segments, as compared to segments 32, when the stent is maintained at the predetermined diameter. Segments 30 also have a higher axial flexibility. Conversely, segments 32 undergo less axial shortening when stent 26, upon its release from a deployment device, radially self-expands. Thus, in stent 26 the end segments and central segment provide the greater stent fixation force, yet more readily conform to any curvature of the vessel in which stent 26 is implanted. This configuration is particularly useful in cases where occlusions are present in more severely curved regions of the vessel.

Figure 5 shows an alternative embodiment stent 34 which, like stent 26, is formed of helically wound and interbraided flexible and biocompatible strands 36. The stent provides an alternating sequence of stent segments 38 with a strand crossing angle of 130 degrees, and stent segments 40 with a strand crossing angle of 150 degrees. In addition, segments 40 are formed to have larger diameters than segments 38 when stent 34 is in the free state.

In Figure 6, stent 34 is shown after its deployment within a vessel 42 defined by a tissue wall 44. Although vessel 42 actually is curved, it is shown in straight lines in Figure 6 to draw attention to the manner in which stent 34 is radially compressed and thereby maintained at a predetermined diameter. In particular, segments 40, while larger than segments 38 in the free state, are radially compressed to the predetermined diameter along with segments 38. Accordingly, as compared to segments 30 of stent 26 and assuming other structural parameters are equal, segments 40 exert a higher level of radially outward force to fix the stent and enlarge an obstructed region of the vessel. As before, segments 40 of stent 34 have higher axial flexibility, and thus more readily conform to vessel curvature.

Figure 7 illustrates another alternative embodiment stent 46 formed of flexible, helically wound and interbraided strands 48. The strands are wound to provide a strand crossing angle that remains substantially constant over the entire stent length, so that alternating stent segments 50 and 52 have the same strand crossing angle.

Segments 50 and 52 are distinguished from one another, based on the makeup of the strands. In particular, along segments 50, each of the strands incorporates two filaments, indicated at 54 and 56. Along segments 52, however, each strand incorporates only filament 54. With filaments 54 and 56 contributing to both the radial force and axial stiffness along segments 50, these segments exert higher levels of radial force, and have higher levels of axial stiffness, as compared to segments 52.

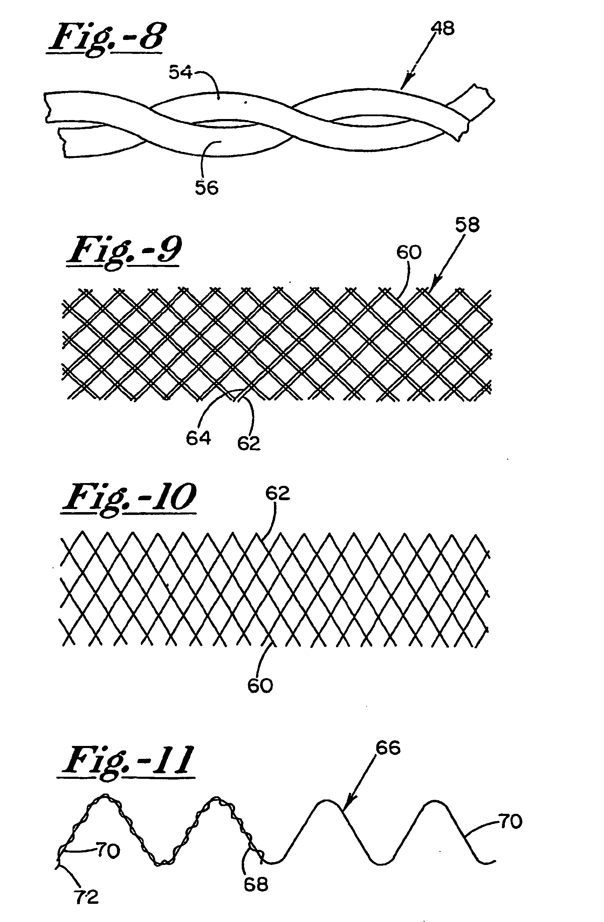

Along segments 50, filaments 54 and 56 may simply be provided side-by-side, and braided in a one pair over-one pair under pattern. In more preferred versions, filaments 54 and 56 are at least slightly twisted into the form of a rope or cable as seen in figure 8. Filaments 54 and 56 can have the same diameter as shown. However, to increase the contrast between the radial force and axial stiffness exhibited by segments 50 as compared to segments 52, filament 54 can have a larger diameter then filament 56. To reduce the contrast, filament 56 can be provided with the larger diameter. Similar results may achieved by providing more than two filaments in each strand. For example, if the strand along segments 50 is composed of three filaments, the strand along segments 52 can consist of either one or two of the filaments, for a greater or lesser contrast between alternating segments.

A wide latitude of control over stent characteristics is afforded by selecting filament materials. In the version of stent 46 shown in figures 7 and 8, both filaments are wires, formed for example of the previously mentioned Elgiloy alloy or stainless spring steel. In another version of this stent, both filaments are formed of a polymer such as PET.

Yet another alternative is to employ filaments formed of a bioabsorbable material from which flexible filaments can be formed. Suitable materials include poly (alpha-hydroxy acid) such as polylactide [poly-L-lactide (PLLA), poly-D-lactide (PDLA)], polyglycolide (PGA), polydioxanone, polycaprolactone, polygluconate, polylactic acid-polyetholene oxide copolymers, poly (hydroxybutyrate), polyanhydride, polyphosphoester, poly(amino acid), and related copolymers. These materials have characteristic degradation rates in vivo. PGA is bioabsorbed relatively quickly, for example in a matter of weeks to months. By contrast, PLA is bioabsorbed over a period of months to years.

In any event, the strands preferably incorporate both bioabsorbable and biostable filaments. Once a stent employing these filaments is implanted, the bioabsorbable filaments begin to degrade, whereby the radial force of the stent and the axial stiffness of the stent gradually decrease in situ, eventually to a point where the stent radial force and axial stiffness, along all segments, is equivalent to the radial force and axial stiffness of a stent incorporating only the biostable filaments. In one specific example of this version of stent 46, filament 54 is formed of PET (Dacron), while filament 56 is formed of one of the bioabsorbable materials mentioned above.

In further versions of stent 46, filament 54 is a wire formed of a metal such as the Elgiloy alloy, and filament 56 is formed of a polymer such as PET.

In general, using different materials to form filaments 54 and 56 enables fabrication of stent 46 using processes other than those available when all filaments are the same kind. In all of the prostheses, strands are formed first by winding all of the intended filaments into strands, after which the strands are inter braided to form an intermediate tubular structure in which each strand is uniform over its entire length. Then, segments 52 are formed by selectively removing portions of filament 56 (or filaments 56 in the case of multiple-filament strands) along a region of the stent corresponding to segments 52. When filaments 54 and 56 are the same material, this selective removal is accomplished by cutting each filament 56 at selected points along the stent length to separate the portions of each filament 56 intended for removal. The cutting and removal is labor-intensive and costly.

When filament 54 is metal and filament 56 is polymeric, the polymeric filaments are susceptible to heat treatments or chemical treatments that have negligible impact on the metallic filaments. Accordingly, the polymeric filaments can be removed by laser ablation or other exposure to heat, or dissolved, as explained in greater detail below.

Figure 9 shows a further alternative embodiment stent 58 formed by helically winding and interbraiding multiple flexible biocompatible strands 60. Each of the strands is composed of two filaments: a filament 62 formed of a biostable polymer such as PET; and a filament 64 formed of a bioabsorbable polymer. Stent 58 is particularly useful in circumstances where the physician desires to implant a stent that initially exerts a high radial force and has a high axial stiffness, but also expects that after a predetermined time, e.g. about a month, the stricture will be remodeled and the initial radial force and axial stiffness levels will no longer be necessary. Once stent 58 is implanted, its radial force and axial stiffness diminish gradually in vivo, due to the degradation of the bioabsorbable polymer. Eventually, substantially all of bioabsorbable filaments 64 are absorbed, leaving a stent 58 composed of strands 60 consisting essentially of filaments 62, as seen in figure 10. Another important advantage is that the pitch or strand crossing angles can be kept low, reducing the amount of axial contraction as the stent radially expands during deployment from the catheter.

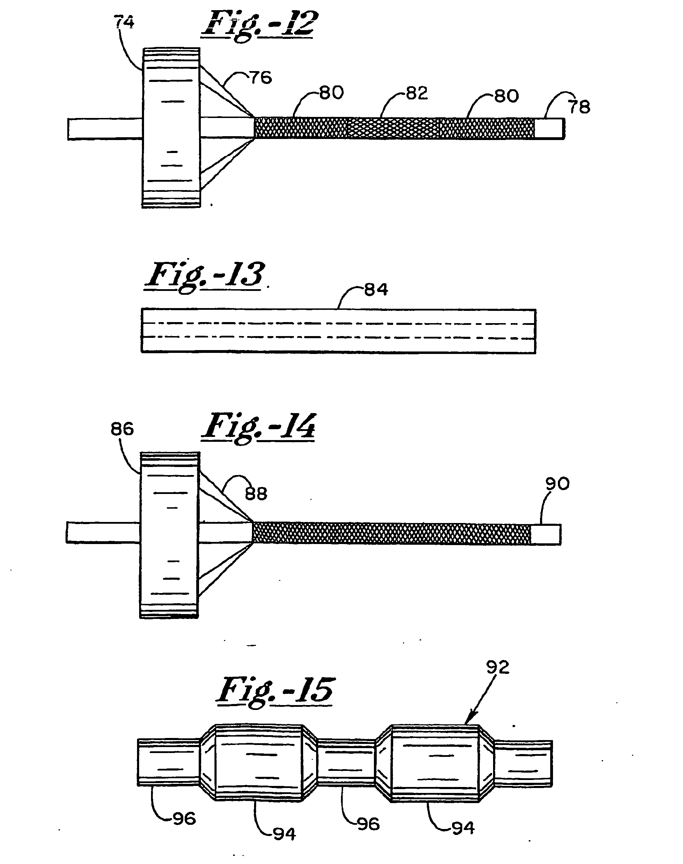

Figure 11 illustrates a further alternative embodiment stent 66, formed of a single, helically wound strand 68. Along a first segment of the stent, the strand incorporates filaments 70 and 72, preferably twisted in the form of a cable or rope as illustrated in figure 8. Along a second segment of the stent, strand 68 incorporates only filament 70. Filament 70 can be formed of metal, or a biostable polymer. Filament 72 can be formed of a metal, a biostable polymer, or a bioabsorbable polymer. Strand 66 can incorporate multiple filaments 70 and multiple filaments 72, if desired.

A variety of processes can be employed for fabricating stents with axial stiffness levels and radial stiffness levels that vary selectively along the stent length. Figure 12 illustrates a braiding apparatus 74 used to simultaneously wind and interbraid a plurality of strands 76 onto a shaping mandrel 78. While just a few strands are illustrated, an exemplary process can involve 36 strands, i.e. 36 separate bobbins (not shown) from which the strands are played out simultaneously.

During winding, the helical pitch of the strands is changed periodically to provide an intermediate tubular structure with segments such as shown at 80 in which the strand crossing angle is 150 degrees, alternating with segments 82 having a strand crossing angle of 130 degrees.

The tubular structure is removed from shaping mandrel 78, and placed over a tubular heat-set mandrel 84 (figure 13), which has a constant diameter, preferably the same as the diameter of the shaping mandrel. Mandrel 84 is heated sufficiently to raise the temperature of the tubular structure at least to a heat-set temperature to thermally impart the desired shape, resulting in a device like stent 26 (figure 4). Table 1 lists structural characteristics for a stent of this type, in which 36 Elgiloy strands, each having a diameter of 0.17 mm, are wound on a 22 mm diameter shaping mandrel and thermally formed on a 22 mm diameter heat-set mandrel. The pressure and stiffness levels in Table 1 characterize the stent when radially compressed to a diameter of 20 mm.

**TABLE 1**

| Strand Crossing Angle, Degrees | Radial Pressure, Pa | Longitudinal Stiffness, N/m |
|---|---|---|
| 50 | 14 | 34.1 |
| 70 | 60 | 30.1 |
| 100 | 314 | 22.6 |
| 125 | 886 | 16.4 |
| 130 | 1,280 | 14.3 |
| 135 | 1,388 | 13.4 |
| 140 | 2,138 | 10.5 |
| 150 | 2,992 | 8.6 |
| 155 | 3,198 | 7.5 |

Table 1 illustrates the general point that stent segments wound at higher strand crossing angles have higher axial flexibility (i.e. lower longitudinal stiffness) and exert higher levels of radially outward force when radially compressed. With particular attention to stent 26, stent segments 30 wound at 150 degrees, as compared to stent segments 32 wound at 130 degrees, exert more than twice the radially outward force (2,992 Pa compared to 1,280 Pa) and have slightly over half the longitudinal stiffness. A tolerance of 5 degrees in the strand crossing angle results in considerable ranges of levels encompassing the nominal radial pressure in particular, but also the nominal longitudinal stiffness.

The present invention encompasses alternative processes. Figures 14 and 15 relate to a process for fabricating stent segments with different strand crossing angles in which the control parameters of the braiding equipment remain constant. As a result, the initial braided structure has a constant strand crossing angle over its complete length. More particularly, Figure 14 shows a braiding apparatus 86 used to simultaneously wind and interbraid a plurality of strands 88 onto a constant-diameter shaping mandrel 90. The resulting intermediate tubular structure has a constant strand crossing angle.

The tubular structure is removed from the shaping mandrel and placed onto a heat-set mandrel that does not have a constant diameter, such as a heat-set mandrel 92 shown in Figure 15, with larger-diameter sections 94 alternating with smaller-diameter sections 96. With the tubular structure surrounding mandrel 92, its opposite ends are pulled away from one another to radially contract the structure and draw it into a closer engagement with mandrel 92. Then, clamps (not shown) are closed against the tubular structure, causing it to more closely conform to the contour of the mandrel.

In one specific example, shaping mandrel 90 has a diameter of 23 millimeters, sections 94 of the heat-set mandrel have a diameter of 23 millimeters, and sections 96 of the heat-set mandrel have a diameter of 22 millimeters. The strand crossing angle of the tubular structure is 150 degrees. When drawn around and clamped against the heat-set mandrel, portions of the tubular structure along mandrel sections 94 assume substantially the same diameter at which the structure was shaped. As a result, the strand crossing angle remains at about 150 degrees. Along smaller mandrel sections 96, the tubular structure is contracted to a smaller diameter of 22 millimeters, with a result that the strand crossing angel is reduced to about 130 degrees.

With the tubular structure conforming to the heat-set mandrel, heat from the mandrel raises the structure temperature at least to a heat-set temperature, whereby the mandrel shape is thermally imparted to the structure. The result is a stent similar to stent 34 (Figure 5), with three smaller-diameter stent segments 38 with a strand crossing angle of 130 degrees, and two larger-diameter stent segments 40 with a strand crossing angle of 150 degrees.

Table 2 lists various structural parameters for a stent formed of 36 Elgiloy alloy strands having a diameter of 0.17 millimeters, for a variety of different mandrel diameters and strand crossing angles. Table 2 illustrates the impact upon crossing angle when the structure is drawn to a reduced diameter, and also indicates resulting pressure and stiffness levels.

**TABLE 2**

| Shaping Mandrel Diameter, mm | Initial Crossing Angle, Degrees | Heat-Set Mandrel Diameter, mm | Post Heat-Set Crossing Angle, Degrees | Radial Pressure, Pa | Longitudinal Stiffness, N/m |
|---|---|---|---|---|---|
| 24 | 130 | 22 | 112 | 561 | 19.3 |
| 24 | 130 | 24 | 130 | 1,239 | 16.1 |
| 25 | 130 | 22 | 106 | 421 | 21 |
| 25 | 130 | 25 | 130 | 1,154 | 17 |
| 24 | 140 | 22 | 119 | 778 | 17.4 |
| 24 | 140 | 24 | 140 | 1,741 | 12.9 |
| 22 | 130 | 22 | 130 | 1,280 | 14.3 |
| 22 | 130 | 21 | 116 | 494 | 17 |
| 23 | 150 | 22 | 135 | 1,595 | 12.8 |
| 23 | 150 | 23 | 150 | 2,679 | 9.2 |

Figure 16 illustrates an alternative embodiment of the preceding process, in which a braiding apparatus 98 is used to wind and interbraid a plurality of strands 100 onto a shaping mandrel 102. Mandrel 102 has a plurality of larger-diameter sections 104 in an alternating sequence with smaller-diameter sections 106. The resulting intermediate tubular structure includes alternating large-diameter segments and smaller-diameter segments. Strands 100 are wound to provide a strand crossing angle that is constant over the length of the intermediate tubular structure. Alternatively, the strands can be wound to provide higher strand crossing angles along the larger-diameter segments, if desired.

According to one version of this process, the intermediate tubular structure is removed from shaping mandrel 102 and disposed about a heat-set mandrel similar to mandrel 92 shown in Figure 15, with alternating larger-diameter and smaller-diameter sections corresponding to the same sections of the shaping mandrel. Sufficient heat is applied to thermally impart the desired shape to the tubular structure, resulting in a stent similar to stent 34 (Figure 5).

According to another version of this process, strands 100 again are wound about shaping mandrel 102, so that the resulting intermediate tubular structure includes larger-diameter segments and smaller-diameter segments in an alternating sequence. Then, however, the intermediate tubular structure is drawn or radially contracted onto a constant diameter heat-set mandrel. Larger-diameter segments of the structure, as compared to smaller-diameter segments, are drawn longitudinally a greater distance to reduce their diameters to the diameter of the heat-set mandrel. Clamps may be used, particularly around the larger-diameter segments, to ensure that the intermediate tubular structure more closely conforms to the mandrel. Accordingly, the resulting constant-diameter stent appears similar to stent 26 in Figure 4, with alternating segments having relatively high and relatively low strand crossing angles. The lower crossing angle segments of the finished stent correspond to the larger-diameter segments of the intermediate tubular structure.

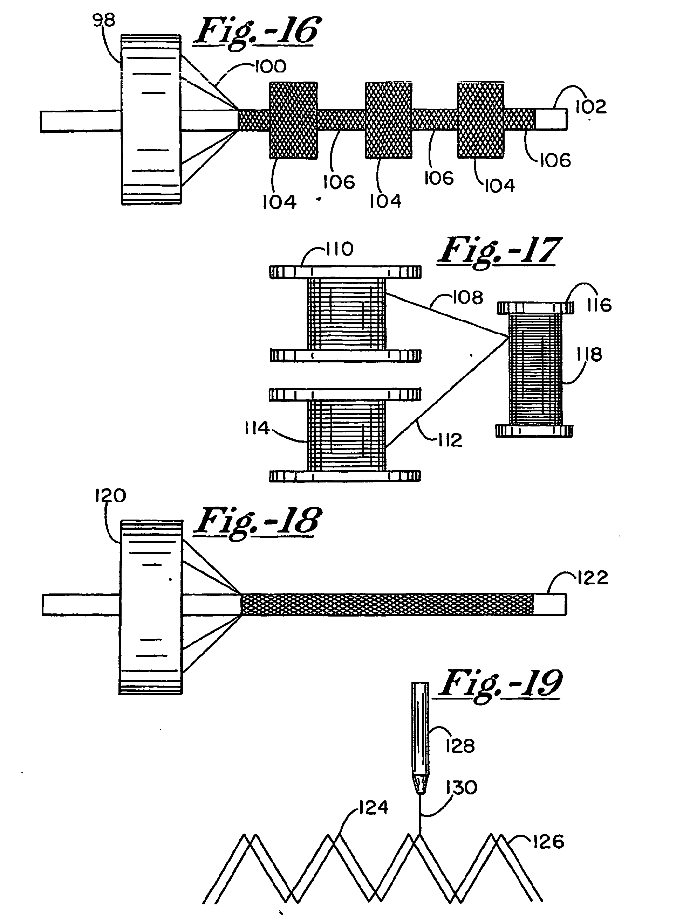

Figures 17 and 18 illustrate initial stages of a process for fabricating a stent similar to stent 46 (Figure 7). In Figure 17, a first filament 108 from a spool 110, and a second filament 112 from a spool 114, are simultaneously wound onto a bobbin 116 to provide a paired-filament strand 118. Bobbin 116 is one of several (e.g. 36) bobbins loaded in a braiding apparatus 120 for simultaneous winding of the paired-filament strands onto a constant-diameter shaping mandrel 122.

In one specific example, each of filaments 108 and 112 is an Elgiloy alloy wire having a diameter of 0.14 mm. A stent formed of paired strands 118 has similar radial force and axial stiffness levels as a stent formed of the same number of single-wire strands at a 0.17 mm diameter, assuming the same strand crossing angle, which in this example is 130 degrees.

In a variant of this process, paired-filament strands, slightly twisted to provide a cable or rope, may be purchased in lieu of performing the bobbin loading step illustrated in Figure 17.

In either event, after braiding the intermediate tubular structure is removed from the shaping mandrel and disposed on a constant-diameter heat-set mandrel preferably having the same diameter as the shaping mandrel, e.g. 22 mm. As before, the intermediate tubular structure is heated to a temperature sufficient to thermally impart the desired shape to the stent.

When removed from the heat-set mandrel, the stent has substantially the same strand crossing angle throughout its length, as well as the same levels of radially outward force and axial flexibility. At this stage, filaments 112 are cut at selected points along the length of the stent, corresponding to junctions between separate stent segments. Following cutting, each filament 112 is severed into alternating first and second filament segments. The first segments are removed from the stent to form segments along which strands 118 include only filaments 108, such as segments 52 of stent 46. The second filament segments remain in place, providing stent segments along which the strand incorporates both filaments 108 and 112.

Table 3 shows levels of radially outward force and axial stiffness in stents employing 36 strands of the Elgiloy alloy. Levels are indicated for the different segments, along which each strand includes two filaments and only one filament, respectively.

**TABLE 3**

| Filament Diameters, mm | Radial Pressure, Pa (2 Wires) | Longitudinal Stiffness, N/m (2 Wires) | Radial Pressure, Pa (1 Wire) | Longitudinal Stiffness, N/m (1 Wire) |
|---|---|---|---|---|
| 0.34 | 40952 | 456 | 20476 | 228 |
| 0.17 | 2560 | 28.6 | 1280 | 14.3 |
| 0.15 | 1552 | 17.4 | 776 | 8.7 |
| 0.12 | 636 | 7.0 | 318 | 3.5 |

Thus, the stent segments along which the strand incorporates two wires, as compared to the stent segments along which the strand has a single wire, exert twice the radially outward force and have twice the longitudinal stiffness, when a stent formed on a 22 diameter mandrel is radially compressed to a 20 mm diameter. As noted above, the two filaments of the strand can have different diameters if desired, to alter the relationship of the alternating stent segments as to both radial force and axial stiffness.

Table 4 shows levels of radially outward force and axial stiffness in stents constructed of a biostable polymer such as PET. Levels are given for stent segments along which the strands consist of single filaments, and for alternate segments in which the strands include two filaments of the given diameter. While not shown in the table, filaments of different diameters would provide cumulative radial pressure and longitudinal stiffness levels when present in the strand. For example, a stent segment along which the strands each incorporate one 0.35 mm diameter filament and one 0.4 mm diameter filament would exert a radial pressure of 1,520 Pa, and have a longitudinal stiffness of 16.6 N/m. In this example, the 36 polymeric strands are wound and interbraided on a 22 mm diameter shaping mandrel at a strand crossing angle of 130 degrees, and heat treated on a heat-set mandrel having the same diameter. As a result, the finished stent has a braid angle of 130 degrees.

**TABLE 4**

| Filament Diameters, mm | Radial Pressure, Pa (2 Filaments) | Longitudinal Stiffness, N/m (2 Filaments) | Radial Pressure, Pa (1 Filament) | Longitudinal Stiffness, N/m (Single Filament) |
|---|---|---|---|---|
| 0.24 | 234 | 2.6 | 117 | 1.3 |
| 0.3 | 586 | 6.4 | 293 | 3.2 |
| 0.35 | 1108 | 12.2 | 554 | 6.1 |
| 0.4 | 1932 | 21 | 966 | 10.5 |
| 0.5 | 4928 | 52.4 | 2464 | 26.2 |

All radial pressure and longitudinal stiffness levels are produced under radial compression of the stent to a diameter of 20 mm. Based on Table 4, a stent segment in which the strands each consist of a pair of polymeric filaments most closely approximates the radial pressure and longitudinal stiffness levels of a stent segment with Elgiloy alloy strands (0.17 mm diameter) when the diameter of each filament is in the range of 0.35 - 0.40 mm.

As previously noted, the paired-filament strands making up stents like stent 46 can consist of filaments formed of different materials: e.g., a filament 54 composed of the Elgiloy alloy, and a filament 56 composed of a biostable polymer such as PET. In such cases stent fabrication is subject to a restriction not present when both filaments are the same. Conversely, certain fabricating options are available that cannot be employed when the filaments are the same.

The restriction applies to the thermal setting stage. In particular, metals like the Elgiloy alloy typically are thermally set at temperatures that not only exceed thermal setting temperatures of the biostable polymers, but also the melting temperatures of the biostable polymers. Accordingly, a braided intermediate tubular structure incorporating both the metal and polymeric filaments cannot simply be heat set on a heat-set mandrel as when all filaments are either metal or polymeric.

To overcome this problem, one alternative process begins with selecting, as the metal for filaments 54, a cobalt-chromium-molybdenum (CoCrMo) alloy containing less than about 5 weight percent nickel as the metallic filament material. Several such alloys are described in U.S. Patent No. 5,891,091 (Stinson), assigned to the assignee of this application. Filaments composed of these alloys can be shaped by cold working, without heat treatment. Accordingly, after an intermediate tubular structure is disposed onto a heat-set mandrel as previously described, the temperature of intermediate structure is heat set by raising it only to the lower heat-set temperature of the polymeric biostable filaments, not to the much higher heat-set temperature of the metal filaments.

According to another suitable alternative process, the metallic filaments are thermally set, and thus tend to assume the desired helical shape, before they are combined with the biostable polymeric filaments. The polymeric filaments may likewise be preshaped, or alternatively may be thermally set after they are combined with the metallic filaments at the much lower heat-set temperatures that apply to the polymer. The preshaping proceeds as described in U.S. Patent No. 5,758,562 (Thompson), assigned to the assignee of this application.

On the other hand, when filaments 54 and 56 are formed of different materials, alternative methods of selectively removing portions of filaments 56 can be employed, which are not available when the filaments are the same. Figure 19 illustrates an intermediate tubular structure 124, following a heat-set stage. Throughout the length of the structure, helically wound and interbraided strands 126 are each composed of a metallic filament and a biostable polymeric filament. The tubular structure is disposed proximate a laser 128 that generates a laser beam 130, either in a continuous wave (CW) or pulsed mode. In either event, beam 130 is caused to selectively impinge upon the polymeric filaments at selected points along the length of the tubular stent.

The selected points can correspond to junctions between high radial force segments and low radial force segments, in which case filaments 56 are severed by laser ablation for later removal from intended low radial force segments. Alternatively, stent 124 can be moved axially relative to the laser beam, such that length portions of filaments 56 along intended low axial stiffness sections are completely removed by laser ablation. In either case, the laser ablation has a negligible impact on adjacent metallic filaments 54. To further ensure that laser ablation removes filaments only along intended lower axial stiffness segments, filaments 56 along the intended higher axial stiffness segments can be masked or shielded during the laser treatment.

The laser ablation processees can be automated, substantially reducing the fabrication cost compared to situations that require cutting the filaments.

In an alternative process, length portions of the polymeric filaments can be dissolved along the intended low axial stiffness segments, using a solution in which the metallic filaments remain stable. Again, it may be desired to mask or shield the polymeric filaments along intended high axial stiffness segments.

In yet another alternative similar to laser ablation, stent 124 is selectively, i.e. specifically along intended lower axial stiffness segments, subjected to heat sufficient to melt the polymeric,filaments. Heat-reflective or heat-insulative shielding can be interposed between adjacent segments, to ensure that the polymeric filaments are melted only along the intended segments.

Thus in accordance with the present invention, stents, stent-grafts and other devices implantable in body vessels can be selectively varied along their lengths both as to axial stiffness and radial force, in each case to achieve an optimum combination of fixation and conformity with body vessel curvature. Alternatively or in addition, such devices can provide high initial levels of radial force and axial stiffness that gradually degrade in vivo. Depending on the fabrication approach, devices can be configured with segments that exceed neighboring segments as to both radial force and axial stiffness, or alternatively exceed neighboring segments as to radial force and axial flexibility. In helically wound devices, pitch and strand crossing angles can be kept lower, to reduce the degree of axial contraction that accompanies radial expansion.

## Claims

1. A prosthesis insertable into body lumens (1) with natural curvature, including:
a body insertable tubular wall (16, 26, 34, 46, 66) incorporating a plurality of first braided tubular wall segments (22, 32, 38, 50, 82) and a plurality of second tubular wall segments (24, 30, 40, 52, 80) in an alternating sequence, the first and second tubular wall segments having respective nominal diameters when in a relaxed state and being radially compressible against an elastic restoring force to a predetermined diameter;
wherein the first and second wall segments when radially compressed to the predetermined diameter have respective axial stiffness levels, with the first tubular wall segments (22, 32, 38, 50, 82) having relatively high first axial stiffness levels, and with the second tubular wall segments (24, 30, 40, 52, 80) having second axial stiffness levels lower than the first axial stiffness levels, whereby the second tubular wall segments (24, 30, 40, 52, 80), as compared to the first tubular wall segments (22, 32, 38, 50, 82), are adapted to more readily conform to a curvature of a body lumen in which the tubular wall is deployed.

2. The prosthesis of claim 1 wherein all of the first axial stiffness levels are substantially the same.

3. The prosthesis of claim 1 wherein all of the second axial stiffness levels are substantially the same.

4. The prosthesis of claim 1 wherein:
the first and second when segments when radially compressed to the predetermined diameter have respective radial force levels, with the first tubular well segments (22, 32, 38, 82) having relatively low first radial force levels, and with the second tubular wall segments (24, 30, 40, 80) having second radial force levels higher than the first radial force levels.

5. The prosthesis of claim 4 wherein all of the first radial force levels are substantially the same, and all of the second radial force levels are substantially the same.

6. The prosthesis of claim 1 wherein:
the body insertable tubular wall (16, 26, 34, 46, 66) is composed of at least one flexible strand (28, 36, 48, 68, 76, 88, 100, 118, 126).

7. The prosthesis of claim 6 wherein:
the at least one flexible strand (28, 36, 48, 68, 76, 88, 100, 118, 126) includes a plurality of flexible strands helically wound in opposite directions to form multiple strand crossings defining strand crossing angles.

8. The prosthesis of claim 7 wherein:
the strand crossing angles along the second tubular wall segments (30, 40, 80) are larger than the strand crossing angles along the first tubular wall segments (32, 38, 82).

9. The prosthesis of claim 7 wherein:
the strand crossing angles along the first tubular wall segments (22, 32, 38, 50, 82) are substantially the same, and the strand crossing angles along the second tubular wall segments (24, 30, 40, 52, 80) are substantially the same.

10. The prosthesis of claim 7 wherein:
the nominal diameters of the second tubular wall segments (40) are larger than the nominal diameters of the first tubular wall segments (38).

11. The prosthesis of claim 10 wherein:
the nominal diameters of the second tubular wall segments are substantially the same, and the nominal diameters of the first tubular wall segments are substantially the same.

12. The prosthesis of claim 7 wherein:
the strand crossing angles along the second tubular wall segments (52) are substantially the same as the strand crossing angles along the first tubular wall segments (50).

13. The prosthesis of claim 6 wherein:
the at least one flexible strand (48, 68) incorporates a first number of filaments along each of the first tubular wall segments and a second number of filaments along each of the second tubular wall segments, wherein the second number is less than the first number.

14. The prosthesis of claim 13 wherein:
the filaments (54, 56, 70, 72) along the first tubular wall segments include first and second different types of filaments.

15. The prosthesis of claim 14 wherein:
the first filament type is selected from the group of filament types consisting of: metallic filaments and biostable non-metallic filaments; and the second filament type is selected from the group of filament types consisting of: metallic filaments, biostable non-metallic filaments, and biodegradable filaments.

16. The prosthesis of claim 15 wherein:
the second type of filament is selected from the group consisting of bioabsorbable filaments.

17. The prosthesis of claim 6 wherein:
the at least one flexible strand (28, 36, 48, 68, 76, 88, 100, 118, 126) includes a plurality of biostable filaments, and a plurality of bioabsorbable filaments.

18. The prosthesis of claim 6 wherein:
the at least one flexible strand (28, 36, 48, 68, 76, 88, 100, 118, 126) includes a first set of flexible filaments (54, 70) spanning substantially the length of the tubular structure and a second set of flexible filaments (56, 72) extending only along the first tubular wall segments.

19. The prosthesis of claim 6 wherein:
the at least one strand (48, 68) comprises a cable incorporating at least two filaments (54, 56, 70, 72) along the first tubular wall segments.

20. The prosthesis of claim 1 wherein:
the tubular wall segments have respective radial force levels when radially compressed to the predetermined diameter, and the radial force levels of the first tubular wall segments (50) are higher than the radial force levels of the second tubular wall segments (52).

21. The prosthesis of claim 20 wherein:
all of the first radial force levels are substantially the same, and all of the second radial force levels are substantially the same.

22. The prosthesis of claim 1 wherein:
the nominal diameters of the first and second tubular wall segments are substantially the same.

23. The prosthesis of claim 1 wherein:
the tubular wall (16, 26, 34, 46, 66) includes end segments at first and second opposite ends thereof, selected from the group of end segments consisting of: two first segments; two second segments; and a first segment and a second segment.

24. The prosthesis of claim 1 wherein:
the first and second tubular wall segments along said alternating sequence are adjacent one another.

25. The prosthesis of claim 1 wherein:
each of the first and second tubular wall segments has an axial length of at least one centimeter.

26. A process for fabricating a prosthesis insertable into body lumens (1) with natural curvature, including:
braiding at least first and second flexible biocompatible and thermally formable strands (28, 36, 48, 68, 76, 88, 100, 118, 126) about a shaping mandrel (90, 102, 122) to form a tubular wall (16, 26, 34, 46, 66);
forming along the tubular wall a plurality of first tubular wall segments (22, 32, 38, 50, 82) and a plurality of second tubular wall segments (24, 30, 40, 52, 80) in an alternating sequence, the first and second segments having respective nominal diameters when in a relaxed state and being radially compressible against an elastic restoring force into a reduced-diameter configuration in which the first and second tubular wall segments have a predetermined diameter, the first tubular wall segments (22, 32, 38, 50, 82) have relatively high first axial stiffness levels, and the second tubular wall segments (24, 30, 40, 52, 80) have second axial stiffness levels lower than the first axial stiffness levels for better conformity to curvature along body lumens; and
while maintaining the tubular wall in a selected shape, heating the tubular wall to a temperature sufficient to thermally impart the selected shape to the tubular wall.

27. The process of claim 26 wherein:
the shaping mandrel (90, 122) has a constant diameter, and braiding the strands about the shaping mandrel includes winding the strands at alternating first and second pitch angles corresponding to the first and second tubular wall segments respectively, wherein the second pitch angles are lower than the first pitch angles.

28. The process of claim 27 wherein:
braiding the strands comprises winding at least first and second flexible biocompatible and thermally formable strands helically about the shaping mandrel in opposite directions to form multiple intersections of the strands.

29. The process of claim 26 further including:
after braiding the strands about the shaping mandrel (90, 102, 122), placing the tubular wall onto a heat-set mandrel (92) having an alternating sequence of first mandrel segments and second mandrel segments with respective first diameters and second diameters larger than the first diameters; and
wherein said heating the tubular wall includes causing the tubular wall to conform to the heat-set mandrel, thereby forming the alternating sequence of first and second tubular wall segments in correspondence to the alternating sequence of the first and second heat-set mandrel segments.

30. The process of claim 29 wherein:
braiding the strands comprises winding first and second flexible biocompatible and thermally formable strands helically about the shaping mandrel in opposite directions to form multiple intersections of the strands.

31. The process of claim 26 wherein:
the shaping mandrel (102) has an alternating sequence of first shaping mandrel segments and second shaping mandrel segments with respective first shaping mandrel diameters and second shaping mandrel diameters less than the first shaping mandrel diameters, and braiding the strands about the shaping mandrel forms the alternating sequence of first and second tubular wall segments in correspondence to the sequence of first and second shaping mandrel segments.

32. The process of claim 31 wherein:
heating the tubular wall comprises causing the tubular wall to conform to a heat-set mandrel having a substantially constant diameter.

33. The process of claim 31 wherein:
heating the tubular wall comprises conforming the tubular wall to a heat-set mandrel having an alternating sequence of first heat-set mandrel segments and second heat-set mandrel segments with respective first heat-set mandrel diameters and second heat-set mandrel diameters less than the first heat-set mandrel diameters.

34. The process of claim 31 wherein:
braiding the strands comprises winding at least first and second flexible biocompatible and thermally formable strands helically in opposite directions about the shaping mandrel to form multiple intersections of the strands.

35. The process of claim 26 wherein:
at least one flexible biocompatible and thermally formable strand (48, 68) includes first and second filaments (54, 56, 70, 72); and
forming the alternating sequence of first and second tubular wall segments comprises selectively removing the second filament (56, 72) along a plurality of predetermined regions of the tubular wall corresponding to the second tubular wall segments.

36. The process of claim 35 wherein:
selectively removing the second filament (56, 72) comprises cutting the second filament at a plurality of selected points along the strand to separate the lengths of the second filament extending along the predetermined regions, then removing the separated lengths of the second filament from the tubular wall (46, 66).

37. The process of claim 35 wherein:
the first filament has a first melting temperature, and the second filament has a second melting temperature lower than the first melting temperature; and
selectively removing the second filament comprises heating the tubular wall at selected points to a temperature higher than the second temperature and lower than the first temperature.

38. The process of claim 37 wherein:
heating the tubular wall at selected points comprises laser ablation of the second filament.

39. The process of claim 35 wherein:
the first filament is substantially insoluble and the second filament is soluble; and
selectively removing the second filament comprises dissolving the second filament along the predetermined regions.

40. The process of claim 35 wherein:
braiding the strands comprises winding the first and second strands helically in opposite directions about the shaping mandrel, wherein each strand incorporates first and second filaments.

## Patentansprüche

1. In Körperlumen (1) einführbare Prothese mit einer natürlichen Krümmung, einschließlich:
einer in einen Körper einführbaren schlauchartigen Wand (16, 26, 34, 46, 66), welche mehrere erste geflochtene Segmente mit schlauchartiger Wand (22, 32, 38, 50, 82) und mehrere zweite Segmente mit schlauchartiger Wand (24, 30, 40, 52, 80) in einer alternierenden Abfolge aufweist, wobei die ersten und zweiten Segmente mit schlauchartiger Wand entsprechende Nenndurchmesser aufweisen, wenn sie sich in einem entspannten Zustand befinden, und radial gegen eine elastische Rückstellkraft auf einen vorbestimmten Durchmesser komprimierbar sind;
wobei die ersten und zweiten Wandsegmente einen entsprechenden axialen Steifigkeitsgrad aufweisen, wenn sie radial auf den vorbestimmten Durchmesser komprimiert werden, wobei die ersten Segmente mit schlauchartiger Wand (22, 32, 38, 50, 82) einen relativ hohen ersten axialen Steifigkeitsgrad aufweisen und wobei die zweiten Segmente mit schlauchartiger Wand (24, 30, 40, 52, 80) einen zweiten axialen Steifigkeitsgrad aufweisen, der niedriger ist als der erste axiale Steifigkeitsgrad, wobei die zweiten Segmente mit schlauchartiger Wand (24, 30, 40, 52, 80) im Vergleich zu den ersten Segmente mit schlauchartiger Wand (22, 32, 38, 50, 82) angepasst sind, um sich leichter an eine Krümmung eines Körperlumens anzupassen, in welchem die schlauchartige Wand angelegt ist.

2. Prothese nach Anspruch, 1, wobei alle der ersten axialen Steifigkeitsgrade im Wesentlichen gleich sind.

3. Prothese nach Anspruch 1, wobei alle der zweiten axialen Steifigkeitsgrade im Wesentlichen gleich sind.

4. Prothese nach Anspruch 1, wobei:
die ersten und zweiten Segmente eine entsprechende Radialkraftstärke aufweisen, wenn sie radial auf den vorbestimmten Durchmesser komprimiert werden, wobei die ersten Segmente mit schlauchartiger Wand (22, 32, 38, 50, 82) eine relativ niedrige erste Radialkraftstärke aufweisen und die zweiten Segmente mit schlauchartiger Wand (24, 30, 40, 52, 80) eine zweite Radialkraftstärke aufweisen, die größer ist als die erste Radialkraftstärke.

5. Prothese nach Anspruch 4, wobei alle ersten Radialkraftstärken im Wesentlichen gleich sind und alle zweiten Radialkraftstärken im Wesentlichen gleich sind.

6. Prothese nach Anspruch 1, wobei:
die in einen Körper einführbare schlauchartige Wand (16, 26, 34, 46, 66) aus mindestens einem flexiblen Strang (28, 36, 48, 68, 76, 88, 100, 118, 126) zusammengesetzt ist.

7. Prothese nach Anspruch 6, wobei:
der mindestens eine flexible Strang (28, 36, 48, 68, 76, 88, 100, 118, 126) mehrere flexible Stränge umfasst, die helikal in entgegen gesetzten Richtungen gewunden sind, um viele Strangüberkreuzungen auszubilden, welche strangüberkreuzende Winkel definieren.

8. Prothese nach Anspruch 7, wobei:
die strangüberkreuzenden Winkel entlang der zweiten Segmente mit schlauchartiger Wand (30, 40, 80) größer sind als die strangüberkreuzenden Winkel entlang der ersten Segmente mit schlauchartiger Wand (32, 42, 82).

9. Prothese nach Anspruch 7, wobei:
die strangüberkreuzenden Winkel entlang der ersten Segmente mit schlauchartiger Wand (22, 32, 38, 50, 82) im Wesentlichen gleich sind und die strangüberkreuzenden Winkel entlang der zweiten Segmente mit schlauchartiger Wand (24, 30, 40, 52, 80) im Wesentlichen gleich sind.

10. Prothese nach Anspruch 7, wobei:
die Nenndurchmesser der zweiten Segmente mit schlauchartiger Wand (40) größer sind als die Nenndurchmesser der ersten Segmente mit schlauchartiger Wand (38).

11. Prothese nach Anspruch 10, wobei:
die Nenndurchmesser der zweiten Segmente mit schlauchartiger Wand im Wesentlichen gleich sind und die Nenndurchmesser der ersten Segmente mit schlauchartiger Wand im Wesentlichen gleich sind.

12. Prothese nach Anspruch 7, wobei:
die strangüberkreuzenden Winkel entlang der zweiten Segmente mit schlauchartiger Wand (52) im Wesentlichen gleich sind wie die strangüberkreuzenden Winkel entlang der ersten Segmente mit schlauchartiger Wand (50).

13. Prothese nach Anspruch 6, wobei:
der mindestens eine flexible Strang (48, 68) eine erste Anzahl von Filamenten entlang jedem der ersten Segmente mit schlauchartiger Wand und eine zweite Anzahl von Filamenten entlang jedem der zweiten Segmente mit schlauchartiger Wand aufweist, wobei die zweite Anzahl kleiner ist als die erste Anzahl.

14. Prothese nach Anspruch 13, wobei:
die Filamente (54, 56, 70, 72) entlang den ersten Segmente mit schlauchartiger Wandn erste und zweite unterschiedliche Typen von Filamenten aufweisen.

15. Prothese nach Anspruch 14, wobei:
der erste Filamenttyp aus der Gruppe von Filamenttypen ausgewählt ist, die besteht aus: metallischen Filamenten und biologisch stabilen nicht metallischen Filamenten, und der zweite Filamenttyp aus der Gruppe von Filamenttypen ausgewählt ist, die besteht aus: metallischen Filamenten, biologisch stabilen, nicht metallischen Filamenten und biologisch abbaubaren Filamenten.

16. Prothese nach Anspruch 15, wobei:
der zweite Filamenttyp aus der Gruppe ausgewählt ist, die aus biologisch resorbierbaren Filamenten besteht.

17. Prothese nach Anspruch 6, wobei:
der mindestens eine flexible Strang (28, 36, 48, 68, 76, 88, 100, 118, 126) mehrere biologisch stabile Filamente und mehrere biologisch resorbierbare Filamente aufweist.

18. Prothese nach Anspruch 6, wobei:
der mindestens eine flexible Strang (28, 36, 48, 68, 76, 88, 100, 118, 126) einen ersten Satz von flexiblen Filamenten (54, 70) aufweist, die im Wesentlichen die Länge der schlauchartigen Struktur umspannen, und einen zweiten Satz von flexiblen Filamenten (56, 72) aufweist, die nur entlang der ersten Segmente mit schlauchartiger Wandn verlaufen.

19. Prothese nach Anspruch 6, wobei:
der mindestens eine Strang (48, 68) ein Kabel aufweist, das mindestens zwei Filamente (54, 56, 70, 72) entlang den ersten Segmenten mit schlauchartiger Wand aufweist.

20. Prothese nach Anspruch 1, wobei:
die Segmente mit schlauchartiger Wand entsprechende Radialkraftstärken aufweisen, wenn sie radial auf den vorbestimmten Durchmesser komprimiert werden, und die Radialkraftstärken der ersten Segmente mit schlauchartiger Wand (50) sind höher als die Radialkraftstärken der zweiten Segmente mit schlauchartiger Wand (52).

21. Prothese nach Anspruch 20, wobei:
alle der ersten Radialkraftstärken im Wesentlichen gleich sind und alle der zweiten Radialkraftstärken im Wesentlichen gleich sind.

22. Prothese nach Anspruch 1, wobei:
die Nenndurchmesser der ersten und zweiten Segmente mit schlauchartiger Wand im Wesentlichen gleich sind.

23. Prothese nach Anspruch 1, wobei:
die schlauchartige Wand (16, 26, 34, 46, 66) Endsegmente an ersten und zweiten gegenüber liegenden Enden davon aufweist, welche aus der Gruppe von Endsegmenten ausgewählt sind, die besteht aus: zwei ersten Segmenten, zwei zweiten Segmenten und einem ersten und einem zweiten Segment.

24. Prothese nach Anspruch 1, wobei:
die ersten und zweiten Segmente mit schlauchartiger Wand entlang der alternierenden Abfolge nebeneinander liegen.

25. Prothese nach Anspruch 1, wobei:
jedes der ersten und zweiten Segmente mit schlauchartiger Wand eine Achsenlänge von mindestens einem Zentimeter aufweisen.

26. Verfahren zum Fertigen einer in Körperlumen einführbaren Prothese (1) mit natürlicher Krümmung, einschließlich:
Flechten von mindestens ersten und zweiten flexiblen biologisch verträglichen und thermisch formbaren Strängen (28, 26, 48, 68, 76, 88, 100, 118, 126) um einen Modellierungsdorn (90, 102, 122), um eine schlauchartige Wand (16, 26, 34, 46, 66) auszubilden;
Formen entlang der schlauchartigen Wand mehrerer erster Segmente mit schlauchartiger Wand (22, 32, 38, 50, 82) und mehrerer zweiter Segmente mit schlauchartiger Wand (24, 30, 40, 52, 80) in einer alternierenden Abfolge, wobei die ersten und zweiten Segmente entsprechende Nenndurchmesser aufweisen, wenn sie sich in einem entspannten Zustand befinden, und radial gegen eine elastische Rückstellkraft zu einer Konfiguration mit reduziertem Durchmesser komprimierbar sind, in welcher die ersten und zweiten Segmente mit schlauchartiger Wand einen vorbestimmten Durchmesser aufweisen, wobei die ersten Segmente mit schlauchartiger Wand (22, 32, 38, 50, 82) einen relativ hohen ersten axialen Steifigkeitsgrad aufweisen und wobei die zweiten Segmente mit schlauchartiger Wand (24, 30, 40, 52, 80) einen zweiten axialen Steifigkeitsgrad aufweisen, der niedriger ist als der erste axiale Steifigkeitsgrad, um sich leichter an eine Krümmung entlang von Körperlumen anzupassen, und
Erhitzen der schlauchartigen Wand, während die schlauchartige Wand in einer ausgewählten Form gehalten wird, auf eine Temperatur, die ausreichend ist, um der schlauchartigen Wand die ausgewählte Form durch Wärmeeinwirkung zu verleihen.

27. Verfahren nach Anspruch 26, wobei:
der Modellierungsdorn (90, 122) einen konstanten Durchmesser aufweist, und Flechten der Stränge um den Modellierungsdorn das Winden der Stränge in alternierenden ersten und zweiten Kantenwinkeln, welche respektive den ersten und zweiten Segmenten mit schlauchartiger Wand entsprechen, umfasst, wobei die zweiten Kantenwinkel kleiner sind als die ersten Kantenwinkel.

28. Verfahren nach Anspruch 27, wobei:
Flechten der Stränge das Winden mindestens erster und zweiter flexibler biologisch verträglicher und thermisch formbarer Stränge helikal um den Modellierungsdorn in entgegen gesetzter Richtung umfasst, um viele Kreuzungen der Stränge zu bilden.

29. Verfahren nach Anspruch 26, des Weiteren umfassend:
Platzieren der schlauchartigen Wand auf einen Hitzefixierungsdorn (92), der eine alternierende Abfolge erster Dornsegmente und zweiter Dornsegmente mit entsprechenden ersten Durchmessern und zweiten Durchmessern, die größer als die ersten Durchmesser sind, aufweist, nachdem die Stränge um den Modellierungsdorn (90, 102, 122) geflochten worden sind; und
wobei das Erhitzen der schlauchartigen Wand bewirkt, dass sich die schlauchartige Wand an den Hitzefixierungsdorn anpasst, wodurch die alternierende Abfolge erster und zweiter Segmente mit schlauchartiger Wand entsprechend der alternierenden Abfolge erster und zweiter Hitzefixierungsdornsegmente ausgebildet wird.

30. Verfahren nach Anspruch 29, wobei:
Flechten der Stränge das Winden mindestens erster und zweiter flexibler biologisch verträglicher und thermisch formbarer Stränge helikal um den Modellierungsdorn in entgegen gesetzter Richtung umfasst, um viele Kreuzungen der Stränge zu bilden.

31. Verfahren nach Anspruch 26, wobei:
der Modellierungsdorn (102) eine alternierende Abfolge erster Modellierungsdornsegmente und zweiter Modellierungsdornsegmente mit entsprechenden ersten Modellierungsdorndurchmessern und zweiten Modellierungsdorndurchmessern, welche kleiner sind als die ersten Modellierungsdorndurchmesser sind, aufweist, und Flechten der Stränge um den Modellierungsdorn die alternierende Abfolge erster und zweiter Segmente mit schlauchartiger Wand entsprechend der Abfolge erster und zweiter Modellierungsdornsegmente ausbildet.

32. Verfahren nach Anspruch 31, wobei:
Erhitzen der schlauchartigen Wand bewirkt, dass sich die schlauchartige Wand an einen Hitzefixierungsdorn mit einem im Wesentlichen konstanten Durchmesser anpasst.

33. Verfahren nach Anspruch 31, wobei:
Erhitzen der schlauchartigen Wand das Anpassen der schlauchartigen Wand an einen Hitzefixierungsdorn mit einer alternierenden Abfolge erster Hitzefixierungsdornsegmente und zweiter Hitzefixierungsdornsegmente mit entsprechenden ersten Hitzefixierungsdorndurchmessern und zweiten Hitzefixierungsdorndurchmessern, welche kleiner sind als die ersten Hitzefixierungsdorndurchmesser sind, umfasst.

34. Verfahren nach Anspruch 31, wobei:
Flechten der Stränge das Winden mindestens erster und zweiter flexibler biologisch verträglicher und thermisch formbarer Stränge helikal um den Modellierungsdorn in entgegen gesetzter Richtung umfasst, um viele Kreuzungen der Stränge zu bilden.

35. Verfahren nach Anspruch 26, wobei:
mindestens ein flexibler biologisch verträglicher und thermisch formbarer Strang (48, 68) erste und zweite Filamente (54, 56, 70, 72) aufweist, und
Formen der alternierenden Abfolge erster und zweiter Segmente mit schlauchartiger Wand das selektive Entfernen des zweiten Filaments (56, 72) entlang mehrerer vorbestimmter Regionen der schlauchartigen Wand entsprechend den zweiten Segmenten mit schlauchartiger Wand umfasst.

36. Verfahren nach Anspruch 35, wobei:
selektives Entfernen des zweiten Filaments (56, 72) das Schneiden des zweiten Filaments an mehreren ausgewählten Punkte entlang des Stranges umfasst, um die Längen des zweiten Filaments zu trennen, welche entlang den vorbestimmten Regionen verlaufen, anschließend Entfernen der abgetrennten Längen des zweiten Filaments von der schlauchartigen Wand (46, 66).

37. Verfahren nach Anspruch 35, wobei:
das erste Filament eine erste Schmelztemperatur aufweist und das zweite Filament eine zweite Schmelztemperatur aufweist, die niedriger ist als die erste Schmelztemperatur, und
selektives Entfernen des zweiten Filaments das Erhitzen der schlauchartigen Wand an ausgewählten Punkten auf eine Temperatur umfasst, die höher ist als die zweite Temperatur und niedriger als die erste Temperatur.

38. Verfahren nach Anspruch 37, wobei:
Erhitzen der schlauchartigen Wand an ausgewählten Punkten Laserablation des zweiten Filaments umfasst.

39. Verfahren nach Anspruch 35, wobei:
das erste Filament im Wesentlichen unlöslich ist und das zweite Filament löslich ist, und
selektives Entfernen des zweiten Filaments das Auflösen des zweiten Filaments entlang den vorbestimmten Regionen umfasst.

40. Verfahren nach Anspruch 35, wobei:
Flechten der Stränge das Winden mindestens erster und zweiter Stränge helikal um den Modellierungsdorn in entgegen gesetzter Richtung umfasst, wobei jeder Strang erste und zweite Filamente beinhaltet.

## Revendications

1. Prothèse insérable dans des lumens corporels (1) à courbure naturelle, comprenant :
une paroi tubulaire insérable dans le corps (16, 26, 34, 46, 66) intégrant une pluralité de premiers segments tressés de paroi tubulaire (22, 32, 38, 50, 82) et une pluralité de deuxièmes segments de paroi tubulaire (24, 30, 40, 52, 80) en séquence alternante, les premiers et deuxièmes segments de paroi tubulaire ayant des diamètres nominaux respectifs lorsqu'ils sont en état relâché et étant compressibles radialement à l'encontre d'une force de restauration élastique jusqu'à un diamètre prédéterminé ;
dans laquelle les premiers et deuxièmes segments de paroi tubulaire, lorsqu'ils sont compressés radialement jusqu'au diamètre prédéterminé, ont des niveaux respectifs de rigidité axiale, les premiers segments de paroi tubulaire (22, 32, 38, 50, 82) ayant de premiers niveaux de rigidité axiale relativement élevés et les deuxièmes segments de paroi tubulaire (24, 30, 40, 50, 82) ayant de deuxièmes niveaux de rigidité axiale inférieurs aux premiers niveaux de rigidité axiale, tandis que les deuxièmes segments de paroi tubulaire (24, 30, 40, 52, 80), comparativement aux premiers segments de paroi tubulaire (22, 32, 38, 50, 82), sont adaptés pour se conformer plus facilement à une courbure d'un lumen corporel dans lequel la paroi tubulaire est déployée.

2. Prothèse selon la revendication 1, dans laquelle tous les premiers niveaux de rigidité axiale sont sensiblement les mêmes.

3. Prothèse selon la revendication 1, dans laquelle tous les deuxièmes niveaux de rigidité axiale sont sensiblement les mêmes.

4. Prothèse selon la revendication 1, dans laquelle :
les premiers et deuxièmes segments, lorsqu'ils sont compressés radialement jusqu'au diamètre prédéterminé, ont des niveaux respectifs de force radiale, les premiers segments de paroi tubulaire (22, 32, 38, 82) ayant de premiers niveaux de force radiale relativement faibles et les deuxièmes segments de paroi tubulaire (24, 30, 40, 80) ayant de deuxièmes niveaux de force radiale plus élevés que les premiers niveaux de force radiale.

5. Prothèse selon la revendication 4, dans laquelle tous les premiers niveaux de force radiale sont sensiblement les mêmes et tous les deuxièmes niveaux de force radiale sont sensiblement les mêmes.

6. Prothèse selon la revendication 1, dans laquelle :
la paroi tubulaire insérable dans le corps (16, 24, 34, 46, 66) est composée d'au moins un toron souple (28, 36, 48, 68, 76, 88, 100, 118, 126).

7. Prothèse selon la revendication 6, dans laquelle :
l'au moins un toron souple (28, 36, 48, 68, 76, 88, 100, 118, 126) englobe une pluralité de torons souples enroulés en spirale dans des sens opposés pour former de multiples croisements de torons définissant des angles de croisement de torons.

8. Prothèse selon la revendication 7, dans laquelle :
les angles de croisement de torons le long des deuxièmes segments de paroi tubulaire (30, 40, 80) sont supérieurs aux angles de croisement de torons le long des premiers segments de paroi tubulaire (32, 38, 82).

9. Prothèse selon la revendication 7, dans laquelle :
les angles de croisement de torons le long des premiers segments de paroi tubulaire (22, 32, 38, 50, 82) sont sensiblement les mêmes et les angles de croisement de torons le long des deuxièmes segments de paroi tubulaire (24, 30, 40, 52, 80) sont sensiblement les mêmes.

10. Prothèse selon la revendication 7, dans laquelle :
les diamètres nominaux des deuxièmes segments de paroi tubulaire (40) sont supérieurs aux diamètres nominaux des premiers segments de paroi tubulaire (38).

11. Prothèse selon la revendication 10, dans laquelle :
les diamètres nominaux des deuxièmes segments de paroi tubulaire sont sensiblement les mêmes et les diamètres nominaux des premiers segments de paroi tubulaire sont sensiblement les mêmes.

12. Prothèse selon la revendication 7, dans laquelle :
les angles de croisement de torons le long des deuxièmes segments de paroi tubulaire (52) sont sensiblement les mêmes que les angles de croisement de torons le long des premiers segments de paroi tubulaire (50).

13. Prothèse selon la revendication 6, dans laquelle :
l'au moins un toron souple (48, 68) intègre un premier nombre de filaments le long de chacun des premiers segments de paroi tubulaire et un deuxième nombre de filaments le long de chacun des deuxièmes segments de paroi tubulaire, le deuxième nombre étant inférieur au premier nombre.

14. Prothèse selon la revendication 13, dans laquelle :
les filaments (54, 56, 70, 72) le long des premiers segments de paroi tubulaire englobent un premier et un deuxième type différent de filaments.

15. Prothèse selon la revendication 14, dans laquelle :
le premier type de filaments est sélectionné parmi le groupe de types de filaments composé de filaments métalliques et filaments non métalliques biostables et le deuxième type de filaments est sélectionné parmi le groupe de types de filaments composé de : filaments métalliques et filaments non métalliques biostables et filaments biodégradables.

16. Prothèse selon la revendication 15, dans laquelle :
le deuxième type de filaments est sélectionné parmi le groupe composé des filaments bioabsorbables.

17. Prothèse selon la revendication 6, dans laquelle :
l'au moins un toron souple (28, 36, 48, 68, 76, 88, 100, 118, 126) englobe une pluralité de filaments biostables et une pluralité de filaments bioabsorbables.

18. Prothèse selon la revendication 6, dans laquelle :
l'au moins un toron souple (28, 36, 48, 68, 76, 88, 100, 118, 126) englobe un premier ensemble de filaments souples (54, 70) sous-tendant sensiblement la longueur de la structure tubulaire et un deuxième ensemble de filaments souples (56, 72) s'étendant seulement le long des premiers segments de paroi tubulaire.

19. Prothèse selon la revendication 6, dans laquelle :
l'au moins un toron (48, 68) comprend un câble intégrant au moins deux filaments (54, 56, 70, 72) le long des premiers segments de paroi tubulaire.

20. Prothèse selon la revendication 1, dans laquelle :
les segments de paroi tubulaire ont des niveaux respectifs de force radiale lorsqu'ils sont compressés radialement jusqu'au diamètre prédéterminé et les niveaux de force radiale des premiers segments de paroi tubulaire (50) sont plus élevés que les niveaux de force radiale des deuxièmes segments de paroi tubulaire (52).

21. Prothèse selon la revendication 20, dans laquelle :
tous les premiers niveaux de force radiale sont sensiblement les mêmes et tous les deuxièmes niveaux de force radiale sont sensiblement les mêmes.

22. Prothèse selon la revendication 1, dans laquelle :
les diamètres nominaux des premiers et deuxièmes segments de paroi tubulaire sont sensiblement les mêmes.

23. Prothèse selon la revendication 1, dans laquelle :
la paroi tubulaire (16, 26, 34, 46, 66) comprend des segments terminaux à ses première et deuxième extrémités, sélectionnés parmi le groupe de segments terminaux composé de : deux premiers segments ; deux deuxièmes segments ; et un premier segment et un deuxième segment.

24. Prothèse selon la revendication 1, dans laquelle :
les premiers et deuxièmes segments de paroi tubulaire le long de ladite séquence alternante sont adjacents les uns aux autres.

25. Prothèse selon la revendication 1, dans laquelle :
chacun des premiers et deuxièmes segments de paroi tubulaire a une longueur axiale d'au moins un centimètre.

26. Procédé de fabrication d'une prothèse insérable dans des lumens corporels (1) à courbure naturelle, comprenant :
le tressage au moins de premiers et deuxièmes torons souples biocompatibles et formables thermiquement (28, 36, 48, 68, 76, 88, 100, 118, 126) autour d'un mandrin de façonnage (90, 102, 122) pour former une paroi tubulaire (16, 26, 34, 46, 66) ;
le formage le long de la paroi tubulaire d'une pluralité de premiers segments de paroi tubulaire (22, 32, 38, 50, 82) et d'une pluralité de deuxièmes segments de paroi tubulaire (24, 30, 40, 52, 80) en séquence alternante, les premiers et deuxièmes segments ayant des diamètres nominaux respectifs lorsqu'ils sont en état relâché et étant radialement compressibles à l'encontre d'une force de restauration élastique jusqu'à une configuration à diamètre réduit dans laquelle les premiers et deuxièmes segments de paroi tubulaire ont un diamètre prédéterminé, les premiers segments de paroi tubulaire (22, 32, 38, 50, 82) ont des premiers niveaux de rigidité axiale élevés et les deuxièmes segments de paroi tubulaire (24, 30, 40, 52, 80) ont des deuxièmes niveaux de rigidité axiale inférieurs aux premiers niveaux de rigidité axiale pour mieux se conformer à la courbure le long de lumens corporels ; et
tout en maintenant la paroi tubulaire dans une forme sélectionnée, le chauffage de la paroi tubulaire à une température suffisante pour donner thermiquement la forme sélectionnée à la paroi tubulaire.

27. Procédé selon la revendication 26, dans lequel :
le mandrin de façonnage (90, 122) a un diamètre constant et le tressage des torons autour du mandrin de façonnage englobe l'enroulement des torons suivant des premiers et deuxièmes angles d'enroulement correspondant respectivement aux premiers et deuxièmes segments de paroi tubulaire, les deuxièmes angles d'enroulement étant inférieurs aux premiers angles d'enroulement.

28. Procédé selon la revendication 27, dans lequel :
le tressage des torons inclut l'enroulement d'au moins des premiers et deuxièmes torons biocompatibles et formables thermiquement en spirale autour du mandrin de façonnage dans des sens opposés pour former des intersections multiples des torons.

29. Procédé selon la revendication 26, incluant en outre :
après le tressage des torons autour du mandrin de façonnage (90, 102, 122), le placement de la paroi tubulaire sur un mandrin à ensemble chauffant (92) ayant une séquence alternante de premiers segments de mandrin et de deuxièmes segments de mandrin avec des premiers diamètres et deuxièmes diamètres respectifs supérieurs aux premiers diamètres ; et
dans lequel ledit chauffage de la paroi tubulaire comprend la mise en conformation de la paroi tubulaire avec le mandrin à ensemble chauffant, ce qui forme la séquence alternante de premiers et deuxièmes segments de paroi en correspondance avec la séquence alternante des premiers et deuxièmes segments de mandrin à ensemble chauffant.

30. Procédé selon la revendication 29, dans lequel :
le tressage des torons comprend l'enroulement de premiers et deuxièmes torons biocompatibles et formables thermiquement en spirale autour du mandrin de façonnage dans des sens opposés pour former des intersections multiples des torons.

31. Procédé selon la revendication 26, dans lequel :
le mandrin de façonnage (102) a une séquence alternante de premiers segments de mandrin de façonnage et de deuxièmes segments de mandrin de façonnage avec des diamètres respectifs de premier mandrin de façonnage et de deuxième mandrin de façonnage inférieurs au diamètre du premier mandrin de façonnage et le tressage des torons autour du mandrin de façonnage forme la séquence alternante de premiers et deuxièmes segments de paroi tubulaire en correspondance avec la séquence des premiers et deuxièmes segments de mandrin de façonnage.

32. Procédé selon la revendication 31, dans lequel :
le chauffage de la paroi tubulaire comprend la mise en conformation de la paroi tubulaire avec un mandrin à ensemble chauffant ayant un diamètre sensiblement constant.

33. Procédé selon la revendication 31, dans lequel :
le chauffage de la paroi tubulaire comprend la mise en conformation de la paroi tubulaire avec un mandrin à ensemble chauffant ayant une séquence alternante de premiers segments de mandrin à ensemble chauffant et de deuxièmes segments de mandrin à ensemble chauffant avec des diamètres de premier mandrin à ensemble chauffant et des diamètres respectif de deuxième mandrin à ensemble chauffant inférieurs aux diamètres du premier mandrin à ensemble chauffant.

34. Procédé selon la revendication 31, dans lequel :
le tressage des torons comprend l'enroulement au moins de premiers et de deuxièmes torons souples biocompatibles et formables thermiquement en spirale autour du mandrin de façonnage dans des sens opposés pour former des intersections multiples des torons.

35. Procédé selon la revendication 31, dans lequel :
au moins un toron souple biocompatible et formable thermiquement (48, 68) englobe de premiers et deuxièmes filaments (54, 56, 70, 72) ; et
le formage de la séquence alternante de premiers et deuxièmes segments de paroi tubulaire comprend l'enlèvement sélectif du deuxième filament (56, 72) le long d'une pluralité de zones prédéterminées de la paroi tubulaire correspondant aux deuxièmes segments de paroi tubulaire.

36. Procédé selon la revendication 35, dans lequel :
le retrait sélectif du deuxième filament (56, 72) comprend le sectionnement du deuxième filament à une pluralité de points sélectionnés le long du toron pour séparer les longueurs du deuxième filament s'étendant sur les zones prédéterminées, puis l'enlèvement des longueurs sectionnées de deuxième filament de la paroi tubulaire (46, 66).

37. Procédé selon la revendication 35, dans lequel :
le premier filament a une première température de fusion et le deuxième filament a une deuxième température de fusion inférieure à la première température de fusion ; et
l'enlèvement sélectif du premier filament comprend le chauffage de la paroi tubulaire à des points sélectionnés jusqu'à une température supérieure à la deuxième température et inférieure à la première température.

38. Procédé selon la revendication 37, dans lequel :
le chauffage de la paroi tubulaire à des points sélectionnés comprend l'ablation au laser du deuxième filament.

39. Procédé selon la revendication 35, dans lequel :
le premier filament est sensiblement insoluble et le deuxième filament est soluble ; et
l'enlèvement sélectif du deuxième filament comprend la dissolution du deuxième filament sur les zones prédéterminées.

40. Procédé selon la revendication 35, dans lequel :
le tressage des torons comprend l'enroulement des premiers et deuxièmes torons en spirale dans des sens opposés autour du mandrin de façonnage, chaque toron englobant des premiers et deuxièmes filaments.
